# EUROPEAN PATENT SPECIFICATION

(11) **EP 3 331 485 B1**
(45) Date of publication and mention of the grant of the patent: **07.04.2021**
(21) Application number: 16900749.9
(22) Date of filing: 30.04.2016
(51) Int. Cl.: A61H 19/00, A61H 37/00, G06F 3/01, G06F 3/0484, G16H 40/67, G06Q 50/00

(54) **INTERACTIVE ONLINE ENTERTAINMENT SYSTEM AND METHOD**
SYSTEM UND VERFAHREN ZUR INTERAKTIVEN ONLINE-UNTERHALTUNG
SYSTÈME ET PROCÉDÉ DE DIVERTISSEMENT EN LIGNE INTERACTIF

(43) Date of publication of application: 13.06.2018
(73) Proprietor: Olivares, Eddy, Guangzhou, Guangdong 510006 (CN); Liu, Dan, Guangzhou, Guangdong (CN)
(72) Inventor: Olivares, Eddy, Guangzhou, Guangdong 510006 (CN); Liu, Dan, Guangzhou, Guangdong (CN)
(74) Representative: Linhart, Friedrich Karl Eberhard
(86) International application number: PCT/US2016/030323
(87) International publication number: WO 2017/189017

(56) References cited:
- US-A1- 2005 027 794
- US-A1- 2005 027 794
- US-A1- 2008 040 146
- US-A1- 2008 040 146
- US-A1- 2009 099 413
- US-A1- 2009 099 413
- US-A1- 2013 116 502
- US-A1- 2013 116 502
- US-A1- 2014 082 495
- US-A1- 2014 082 495
- US-B1- 6 368 268
- US-B1- 6 368 268

## Description

### FIELD OF THE INVENTION

The present invention generally relates to telecommunications. More particularly, the present invention is directed to methods and systems for interactive online adult entertainment.

### BACKGROUND OF THE INVENTION

Chat rooms are widely used to allow two or more users usually located at different locations to communicate. Generally, chat rooms utilize text input by the users that can be displayed in real-time for providing a written transcript of a conversation. Some forms of chatting incorporate video and audio so that two or more users can view some or all of the users from different locations in real-time while conversing.

Some chat rooms provide adult content to provide entertainment for adults. In this regard, existing adult chat rooms generally function similarly to conventional chat rooms. Current challenge for adult chat rooms is to provide the right kinds of interactivity and real world features to provide enjoyable experiences to users and can therefore be attractive to a large number and wide range of users. In this regard, the invention described herein addresses this problem.

US 2009/0099413 A1 describes electronic stroke devices and a system for remote control and interactive play.

### SUMMARY OF THE INVENTION

In view of the disadvantages inherent in the known types of systems for chat rooms and communication systems now present in the prior art, the present invention provides an improved interactive online communication system for adult entertainment. In particular, the invention provides a system for providing online communication according to claim 1 and a corresponding method.

The following discloses a simplified summary of the specification in order to provide a basic understanding of some aspects of the specification. This summary is not an extensive overview of the specification. It is intended to neither identify key or critical elements of the specification nor delineate the scope of the specification. Its sole purpose is to disclose some concepts of the specification in a simplified form as to prelude to the more detailed description that is disclosed later.

Some embodiments include, for example, devices, systems, and methods of providing adult entertainment via online chat sessions and interactive shows.

Some embodiments include a system comprising a server with a memory having stored thereon instructions, and a processor to execute the instructions resulting in a web browser extension or an adult video chatting website for allowing users to interact with models from a distance, for example, by tipping the models during an online video chat session on a website, wherein the models can define tipping parameters to perform predefined acts, via an adult toy or another stimulation device, based on the amount of tip received.

In some embodiments, the web browser extension is to receive tip parameters defining valid tip ranges; and to automatically identify whether a received tip amount is valid based on a criterion related to the tip parameters. If the tip amount is valid, the web browser extension is to communicate with the adult toy to actuate the same. In this regard, the adult toy can be Wi-Fi-enabled to receive commands directly from the server via the web browser extension. Alternatively, the adult toy can be connected via Bluetooth™ (or via another short-range wireless interconnection) to an application installed on a device operated by the model, wherein the application communicates with the web browser extension to relay commands to the adult toy therefrom.

In some embodiments, two or more users can interact with one model across multiple platforms simultaneously via different chat rooms on various websites. In this way, one model can perform online shows and receive tips from multiple users in one or more online chat sessions at the same time.

In some embodiments, the browser extension is configured to generate live control links so as to allow certain users to control the model's adult toy for a limited or unlimited amount of time via a virtual control panel. The browser extension is further configured to automatically cancel previously generated links each time a new link is generated, or automatically cancel expired links. Alternatively, the browser extension can form a queue or add users to a queue of multiple live control links. In this way, multiple users can take turns to control the model's adult toy.

In the light of the foregoing, these and other objects are accomplished in accordance of the principles of the present invention, wherein the novelty of the present invention will become apparent from the following detailed description and appended claims.

### BRIEF DESCRIPTION OF THE DRAWINGS

The above and other objects and advantages of the present invention will be apparent upon consideration of the following detailed description, taken in conjunction with the accompanying exemplary drawings, in which like reference characters refer to like parts throughout, and in which:
FIGs. 1 and 2 show high-level block diagrams of the present system.
FIGs. 3A, 3B, 4, and 5 show exemplary flow charts of the present method.
FIGs. 6 through 9 show exemplary diagrams of various embodiments of the present invention.

### DETAILED DESCRIPTION OF THE INVENTION

The present invention is directed towards a system for providing online communication according to claim 1 and towards a corresponding method. For purposes of clarity, and not by way of limitation, illustrative views of the present system and method are described with references made to the above-identified figures.

As used in this application, the terms "component," "module," "system," "interface," or the like are generally intended to refer to a computer-related entity, either hardware or a combination of hardware and software. For example, a component can be, but is not limited to being, a process running on a processor, an object, and/or a computer. By way of illustration, both an application running on a controller and the controller can be a component. One or more components can reside within a process and/or thread of execution and a component can be localized on one computer and/or distributed between two or more computers. As another example, an interface can include I/O components as well as associated processor, application, and/or API components.

It is to be appreciated that determinations or inferences referenced throughout the subject specification can be practiced through the use of artificial intelligence techniques. In this regard, some portions of the following detailed description are presented in terms of algorithms and symbolic representations of operations on data bits or binary digital signals within a computer memory. These algorithmic descriptions and representations may be the techniques used by those skilled in the data processing arts to convey the substance of their work to others skilled in the art.

Furthermore, the claimed subject matter can be implemented as a method, apparatus, or article of manufacture using standard programming and/or engineering techniques to produce software, firmware, hardware, or any combination thereof to control a computer to implement the disclosed subject matter. The term "article of manufacture" as used herein is intended to encompass a computer program accessible from any computer-readable device, or media.

Discussions herein utilizing terms such as, for example, "processing," "computing," "calculating," "determining," "establishing," "identifying," "analyzing," "checking," or the like, may refer to operations(s) and/or process(es) of a computer, a computing platform, a computing system, or other electronic computing device, that manipulate and/or transfer data represented as physical (e.g., electronic) quantities within the computer's registers and/or memories into other data similarly represented as physical quantities within the computer's registers and/or memories or other information storage medium that may store instructions to perform operations and/or processes.

Moreover, the word "exemplary" is used herein to mean serving as an example, instance, or illustration. Any aspect or design described herein as "exemplary" is not necessarily to be construed as preferred or advantageous over other aspects or designs. Rather, use of the word exemplary is intended to disclose concepts in a concrete fashion. As used in this application, the term "or" is intended to mean an inclusive "or" rather than an exclusive "or."

Additionally, the articles "a" and "an" as used in this application and the appended claims should generally be construed to mean "one or more" or "at least one" unless specified otherwise or clear from context to be directed to a singular form. Similarly, the terms "plurality" and "a plurality" as used herein includes, for example, "multiple" or "two or more." For example, "a plurality of items" includes two or more items. As used herein, the terms "user," "users," "end user," "end users," "audience," "client," "clients," "customer," and "customers" are interchangeable unless the context clearly suggests otherwise. Similarly, as used herein, the terms "model," "models," "performer," "performers," are used interchangeably unless the context clearly suggests otherwise. The terms "web browser extension," "browser extension," and "website" are used interchangeably unless the context clearly suggests otherwise. Furthermore, the foregoing terms "web browser extension," "browser extension," and "website" may be collectively referred to as "application," "software," or "software application."

Some embodiments of the present invention may include one or more wired or wireless links, may utilize one or more components of wireless communication, may utilize one or more methods or protocols of wireless communication, or the like. Some embodiments may utilize wired communication and/or wireless communication.

Some embodiments may be used in conjunction with various devices and systems, for example, a personal computer (PC), a desktop computer, a mobile computer, a laptop, a tablet computer, a server computer, a handheld device, a personal digital assistant (PDA), a wireless communication device, a smart phone, a non-portable device, a wireless access point (AP), a wired or wireless router, a wired or wireless modem, a wired or wireless network, a local area network (LAN), a wireless LAN (WLAN), a metropolitan area network (MAN), a wireless MAN (WMAN), a wide area network (WAN), a wireless WAN (WWAN), a personal area network (PAN), a wireless PAN (WPAN), or networks operating in accordance with existing and/or future versions and/or derivatives of long term evolution (LTE), a device which incorporates a global positioning system (GPS) receiver or transceiver or chip, a device which incorporates an RFID element or chip, a multiple input multiple output (MIMO) transceiver or device, a single input multiple output (SIMO) transceiver or device, a multiple input single output (MISO) transceiver or device, a device having one or more internal antennas and/or external antennas, or the like.

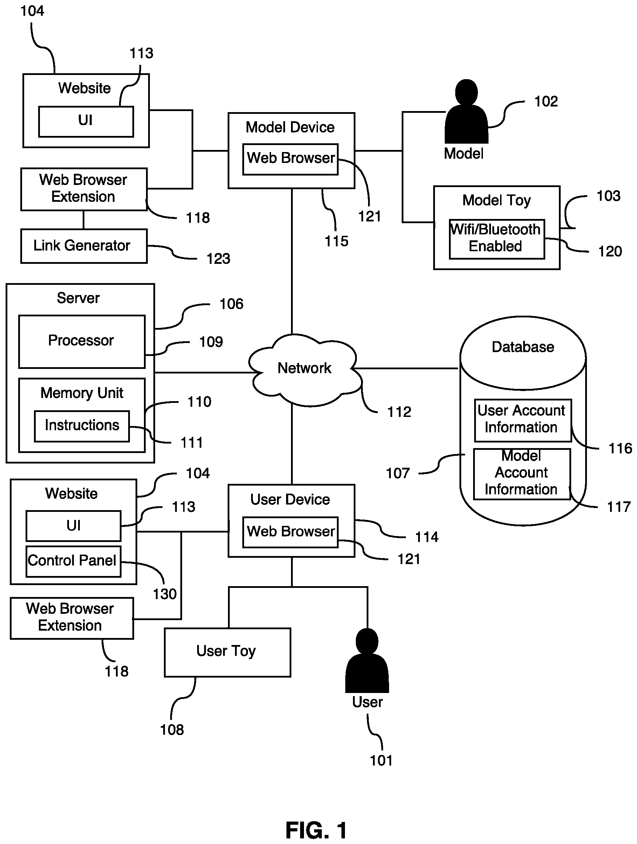

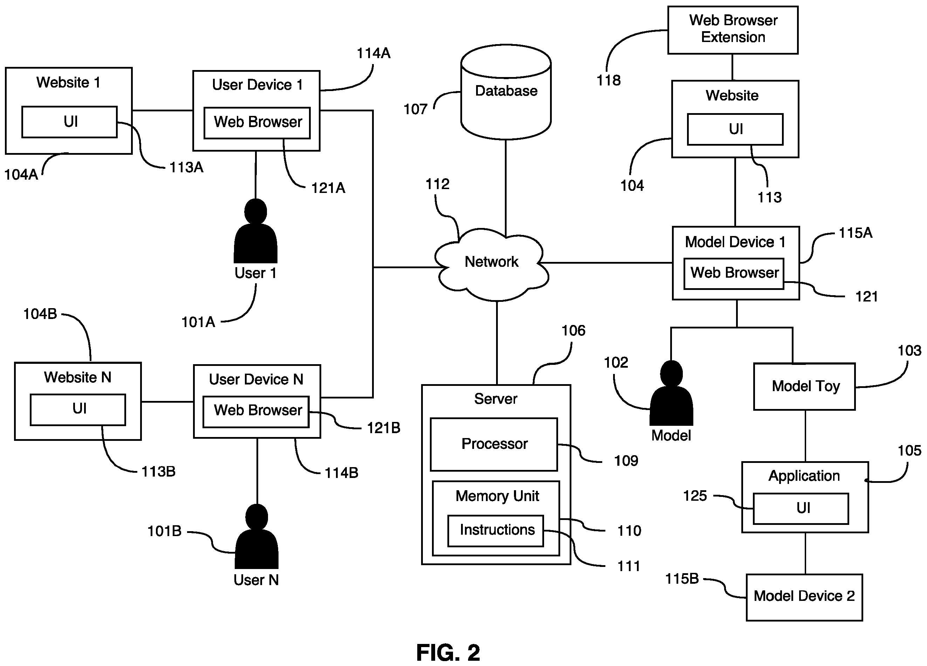

Referring now to **FIGs. 1** and **2****,** there are shown high-level block diagrams of the present system in accordance with some embodiments of the present invention. In one embodiment, the present invention comprises a user device **114** that is operated by a user **101,** wherein the user device **114** comprises a wide variety of computer systems and/or a terminal that allow the user **101** to access a web browser **121** on which the user **101** can access a website **104** to engage in an online chat with a model **102** via a user interface (UI) **113** of the website **104.** In this regard, the device **114** may be implemented using suitable hardware components and/or software components, for example, processors, controllers, memory units, storage units, input units, output units, communication units, operating systems, applications, or the like.

The present system further comprises a model device **115** that is operated by a model **102.** The model device **115** also comprises a variety of computer systems and/or a terminal that allow the model **102** to access a web browser **121** on which the model **102** can access the website **104** to engage in an online chat with the user **101** via a UI **113** of the website **104.** In this regard, the device **114** may be implemented using suitable hardware components and/or software components, for example, processors, controllers, memory units, storage units, input units, output units, communication units, operating systems, applications, or the like.

In some embodiments, the model device **115** comprises an application **105** (e.g., non-downloadable or downloadable mobile application, web application, mobile application) stored thereon. Alternatively, the model **102** comprises a second model device **115B** having the application **105** installed thereon. The application **105** communicates with the model toy **103** so as to send commands thereto (FIG. 2), wherein the model toy **103** comprises various types of adult toys and stimulation devices. It is contemplated that the application **105** comprises a UI **125** for manually controlling the model toy **103,** for example, by transmitting signals to turn the model toy **103** on and off and vibrate the model toy **103.** In other embodiments, however, it is contemplated that the model toy **103** is Wi-Fi or Bluetooth™ enabled **120** (or enabled via other suitable short-range wireless interconnection) so as to allow the model toy **103** to directly communicate with the web browser **121** via the model device **115** (FIG. 1) and receive commands therefrom without the application **105** when the model toy **103** is turned on. In this regard, the model toy **103** is configured to receive signals from the model device **115** and/or the web browser **121**. In some embodiments, the system further comprises a user toy **108** (i.e., Wi-Fi-enabled or Blutooth™ enabled) that can communicate with the web browser extension **118** of the web browser **121** on the user device **114.** It is contemplated that the user toy **108** operates similarly to the model toy **103.**

The website **104** is configured to allow the user **101** and the model **102** to set up a user account and a model account, respectively. In this regard, the user **101** can create his or her user name and password and input payment information, among other types of information associated with the user. Similarly, the model **102** can create a model account by inputting the model's name, age, gender, location, and the like. The user account information **116** and the model account information **117** are stored in a database **107** that is connected to the network **112** of the present system.

The user **101** can select a model **102** from a group of models to enter into an online chat session, via the website **104.** In this regard, the chat session can be a private (i.e., one-on-one) session, a group session (i.e., limited number of users and one model), or a public session (i.e., unlimited number of users). Each model **102** can define and edit tip parameters that are used during each chat session. In one embodiment, the website **104** allows the model to designate specific tip amounts and/or ranges of tip amounts and actions correlating to each of the tip amounts and/or ranges. In this regard, the website **104** is configured to recognize tip amounts and send commands to the toy **103** either directly (if the toy is Wi-Fi enabled **120**) or via the application that is connected to the model toy **103.** In another embodiment, the model can use the web browser extension **118** to input the foregoing parameters. It is noted that as used herein, the terms "action," "actions," "act," and "acts" mean the model's performances, the model's use of the model toy **103** and/or the operation of the model toy **103** (e.g., vibrating, rotating, thrusting, oscillating, etc.). For example, the model **102** can set tip parameters such that tip amounts between 1 to 10 tokens may cause the model toy **103** to vibrate at a low speed for 10 seconds, and tip amounts between 11 to 20 tokens may cause the model toy **103** to thrust at a high speed for 10 seconds. Alternatively, a tip amount of 30 tokens may cause the model toy **103** to rotate at a high speed for 15 seconds.

The UI **113** of the website **104** is an interface between the user **101** and one or more elements of the present system (e.g., the website **104**), the web browser extension **118,** or between the model **102** and one or more elements of the present system. In this regard, the UI **113** of the website **104** allows the user **101** and the model **102** to input and receive messages in a textual format so as to have a live conversation with each other (e.g., in an online chat room). Additionally, the website **104** is further configured to provide a UI **113** comprising audio and video (i.e., during an online video chat session) so that the user **101** can view and listen to the model **102** during a chat session. In some embodiments, the present invention may comprise other downloadable and/or a non-downloadable software application (e.g., a web application, a mobile application) in lieu of the website **104.**

The server **106** comprises a memory unit **110** having instructions **111** stored thereon, and a processor **109,** wherein the processor **109** is configured to execute the instructions **111** resulting in a software such as the web browser extension **118** or the website **104,** wherein the web browser extension **118** or the website **104** is configured to scan for tips during chat sessions and receive tips from the user **101.** The web browser extension **118** or the website **104** can determine whether the received tip falls within one of the tip parameters or whether the tip is equal to a specified tip amount defined by the model **102.** For example, if one of the tip parameters is 1 to 10 tokens to vibrate the model toy **103** at a low speed for 10 seconds, and the user tips 4 tokens, then the tip amount is valid. If, however, one of the tip parameters is 11 to 20 tokens to vibrate the model toy **103** at a high speed for 10 seconds, and the user wishes to vibrate the model toy **103** at a high speed instead of a low speed, the tip amount is not valid and the user may be prompted to adjust (i.e., increase) the tip amount, in some embodiments. Alternatively, the model toy **103** can still react as long as the tip falls in one of the tip parameters. It is contemplated that the user **101** can purchase credits, points, or other types of virtual currency such as tokens that can be credited to his or her account **116** and use the virtual currency to tip the model **102.**

If the web browser extension **118** or the website **104** determines that the received tip falls within one of the tip parameters, it actuates the model toy **103** in accordance with the tip amount. Alternatively, if the web browser extension **118** or the website **104** determines that the received tip falls within one of the tip parameters, it signals the application **105** to actuate the model toy **103.** It is contemplated that the received tip amounts are credited to the model's account **117** and made redeemable by the respective model at a later time. If the web browser extension **118** determines that the received tip does not fall into any of the tip parameters, the web browser extension **118** may be configured to send a notification to the user **101** indicating that the tip amount is insufficient.

In some embodiments, the web browser extension **118** or the website **104** comprises a link generator **123** for generating live control links and transmitting the links to users **101** that are designated by the model **102.** It is contemplated that the link generator **123** comprises a button that can be clicked, tapped, or otherwise activated to automatically generate new live control links, via the server **106,** and cancel previously generated live control links. The model **102** can set parameters to define the duration for which the model **102** would permit the user **102** to control the model toy **103.** In this way, the live control links allow certain users **101** to control the model toy **103** for a limited or an unlimited time, wherein the users **101** can control the model toy **103** via a virtual control panel **130** that is accessible when the live control link is used. The live control link may be available to the user **101** while the user **101** is in a chat session. If more than one user **101** receives a live control link, then the web browser extension **118** or the website **104** creates a queue to add additional links, for example, in the order accessed.

Some embodiments of the present system comprise two or more users **101A, 101B** in communication with one model **102** at the same time, wherein each of the users **101A, 101B** operates a user device **121A, 121B** for accessing the website **104A, 104B** via the web browser **121A, 121B.** In this regard, the first user **101A** and the second user **101B** can use different web browsers so that the first web browser **121A** and the second web browser **121B** need not be the same. Additionally, each user **101A, 101B** can visit different websites to enter into an online chat session with the model 102 such that the first website **104A** and the second website **104B** need not be the same.

In this regard, the model 102 can broadcast on multiple platforms simultaneously (referred hereto as "split-camming") so that each user **101A, 101B** can view the model via, for example, UI **113A, 113B,** and tip the model **102.** One model and two or more users **101A, 101B** can be in a single chat session. When multiple users **101A, 101B** tip the model **102,** the web browser extension **118** or the website **104** can form a queue and build on tips received. For example, the model toy **103** can react to a first tip received, and then react to a second tip received, wherein the first tip is received before the second tip. If the first user **101A** tips the first tip and the second user **101B** tips the second tip, then the each of the users may be notified when the model toy **103** is reacting to their respective tip amounts. Alternatively, one model **102** can be in multiple chat sessions with individual users **101A, 101B** (i.e., one chat session per user). In this regard, the web browser extension **118** can scan all of the websites **104A, 104B** simultaneously for tips to actuate the model toy **103** or to signal the application **105** to make the model toy **103** react based on the received tip amounts.

In some embodiments, one user **101** may enter into multiple chat sessions with multiple models simultaneously. In this regard, the web browser extension **118** for each model **102** can scan for tips designated for that model **102** and actuate the model toy **103** belonging to the respective model **102,** or signal the model's application **105** to actuate the model toy **103.**

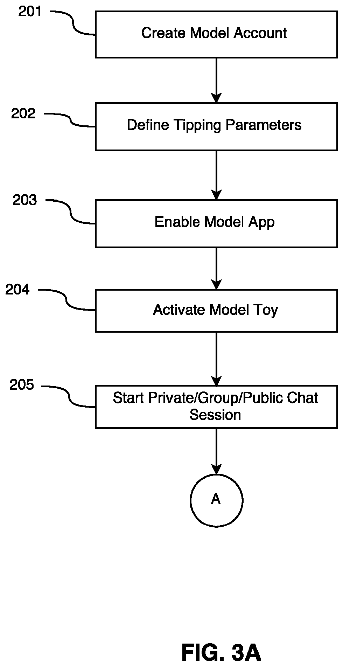

Reference is also made to **FIGs. 3A****,** **3B****,** **4****,** and **5****,** which schematically illustrates exemplary methods of interactive online communication for adult entertainment. In some embodiments, one or more of the operations of **FIGs. 3A** through **5** may be performed by one or more elements of the system (e.g., web browser extension **118** (FIGs. 1, 2)).

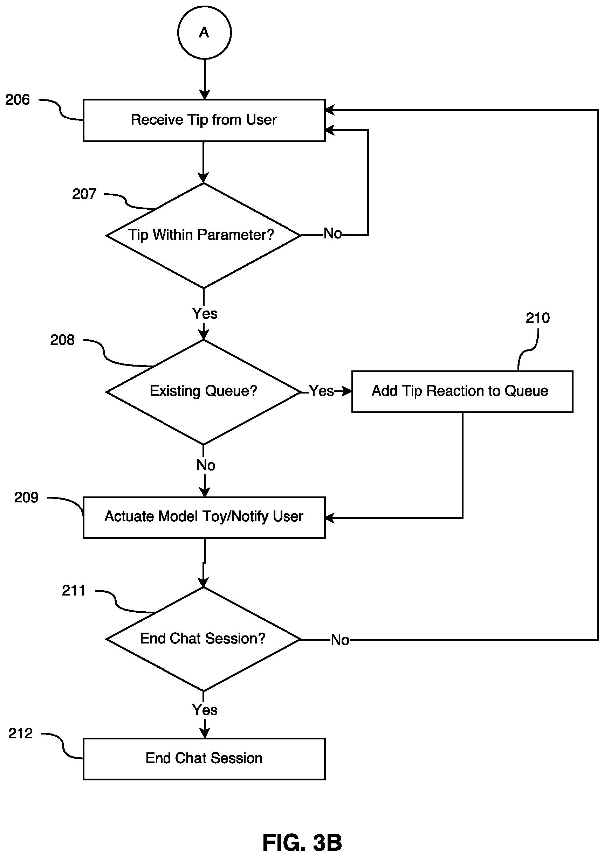

One embodiment for the present method in operation is illustrated in **FIGs. 3A** and **3B****.** As indicated in block **201,** the method may include creating a model account via a website **104** (FIGs. 1, 2), wherein the model account includes information associated with the model such as the model's name, age, gender, location, and the like. As indicated in block **202,** the method may include defining tipping parameters via the website **104** or the web browser extension **118** (FIGs. 1, 2), wherein the tipping parameters can be determined by the model such that the parameters can vary from a model to a model, and wherein the tipping parameters include specific tip amounts or one or more ranges of tip amounts and acts corresponding thereto. For example, tip amounts between 1 to 10 tokens may cause the model toy to vibrate at a low speed for 10 seconds, and tip amounts between 11 to 20 tokens may cause the model toy to vibrate at a high speed for 10 seconds.

Optionally, as indicated in block **203,** the model can enable the application **105** (FIG. 2) stored on the model device **115** (FIGs. 1, 2), if the model toy **103** (FIGs. 1, 2) is not Wi-Fi-enabled. The application **105** (FIGs. 2) can activate the model toy **204** before the model enters into a chat session with a user. If the model toy is Wi-Fi-enabled **120** (FIGs. 1, 2), the application **105** (FIG. 2) may not be needed and the model toy **103** (FIGs. 1, 2) can be activated **204** via a control button (i.e., a power switch or button) disposed on the model toy **103** (FIGs. 1, 2).

As indicated in block **205,** the model can start a chat session (i.e., a private session, a group session, or a public session) with a client via any online chat platform, including a third party platform. The model can receive tips from a client **206** during a chat session. When the model receives a tip, the web browser extension **118** or the website **104** (FIGs. 1, 2) determines whether the tip is within the model's tip parameters **207.** If the tip amount received falls within one of the tip parameters, the web browser extension **118** (FIGs. 1, 2) determines whether there is an existing queue **208.** If there is no existing queue, the model toy is actuated **209** via the application **105** (FIG. 2) of the model device **115** (FIGs. 1, 2). Alternatively, the web browser extension **118** (FIG. 1) may be configured to directly actuate the Wi-Fi-enabled **120** (FIG. 1) model toy **103** (FIG. 1). If there is a queue, the tip reaction is added to the queue **210,** wherein the tip reaction is added in order received.

In some embodiments, if the tip does not fall within any of the tip parameters or meet a specific tip amount, the web browser extension **118** (FIG. 1, 2) can notify the user to indicate that the tip did not fall within the model's tip parameters and that the user needs to adjust (i.e., increase) the tip amount. Alternatively, no actions are taken if the tip amount does not fall within any of the tip parameter. The user can continue tipping the model until the chat session ends.

As indicated in block **211,** if the model indicates that it is the end of a chat session, the chat session is ended **212.** The user may or may not be able to end the chat session. For instance, the user may be able to end the chat session if the chat session is a private session. However, the user may not be able to end the chat session if the chat session is a group session or a public session. Alternatively, the chat session can automatically end **212** if the chat session is valid for only a predetermined period of time.

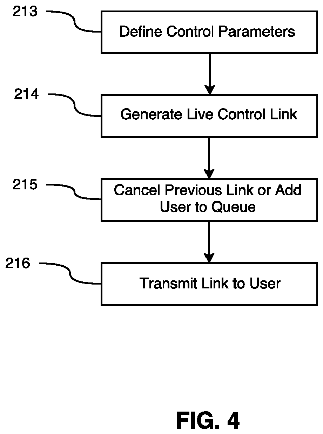

Exemplary steps for generating a live control link are illustrated in **FIG. 4****.** Optionally, the model can generate a live control link via the link generator **123** (FIGs. 1, 2). If the model generates a live control link, the model can define the control parameters (e.g., amount of time a user can control the model toy) as indicated in block **213.** Once the control parameters are defined, the link generator generates a live control link **214** via the server **106** (FIGs. 1, 2). The link generator may automatically cancel or invalidate previously generated links so that only valid links can be activated **215.** Alternatively, the link generator may create a queue if more than one live control links is distributed to multiple users **215.** The generated links are transmitted to users **216** via various messaging systems, for example, by email. The live control link can be used while the user is in a chat session (i.e., a private session, a group session, or a public session) or when the user is not in a chat session.

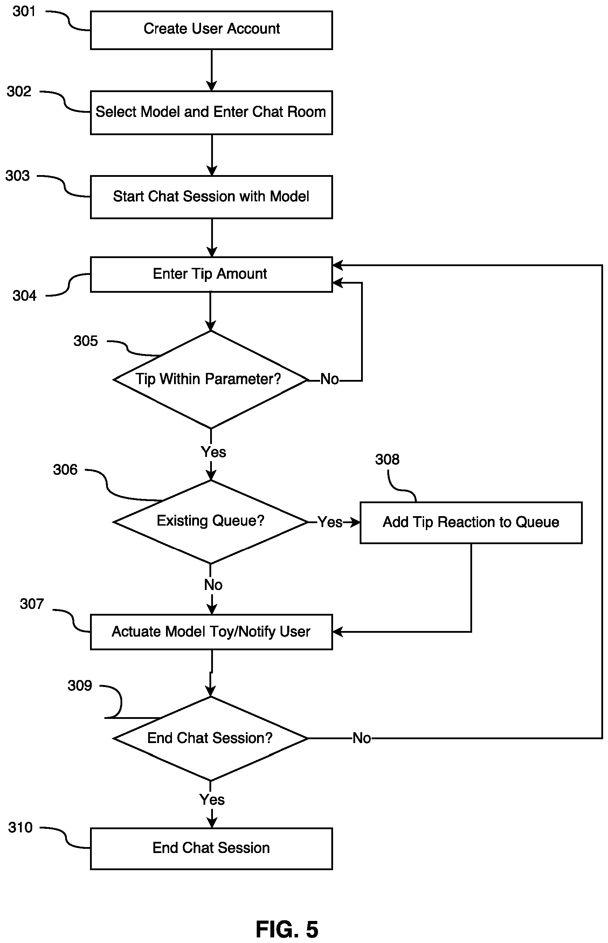

Another embodiment for the present method in operation is illustrated in **FIG. 5****.** As indicated in block **301,** the method further includes creating a user or a client account via a website **104** (FIGs. 1, 2), wherein the user account includes user name and password and input payment information, among other types of information associated with the user. As indicated in block **302,** the user can select a model from a group of models to enter into a chat session and then begin a chat session with a model **303,** wherein the chat session can be private, group, or public. Additionally, it is noted that one or more users may already be in a chat room, the chat room can be empty, or the model and the user can enter the chat room simultaneously.

As indicated in block **304,** the user can enter a tip amount via the UI **113** (FIGs. 1, 2) of the website **104** (FIGs. 1, 2) during the chat session, for example, by typing in the amount of tip within a conversation thread, or by selecting a tip amount from a drop down menu. In some embodiments, the model's tip parameters may be made available to the user during the chat session so that the user can view the tip parameters and tip the model accordingly. In some embodiments, the user can tip the model anonymously, for example, during a group session. As indicated in block **305,** the web browser extension **118** or the website **104** (FIGs. 1, 2) determines whether the user paid the correct tip amount (i.e., a tip amount that falls within one of the tip parameters defined by the model). If the user did not tip the correct amount, the web browser extension **118** (FIGs. 1, 2) can optionally notify the user to adjust the tip amount. Alternatively, the web browser extension **118** may not take any actions until the correct tip amount is input.

As indicated in block **306,** if the correct tip amount is inputted, the web browser extension **118** (FIG. 1) or the website **104** determines whether there is an existing queue **306.** As indicated in block **307,** if there is no existing queue, the web browser extension **118** determines the corresponding predetermined act based on the tip amount and actuates the Wi-Fi-enabled **120** (FIG. 1) model toy **103** (FIG. 1) or the web browser extension **118** or the website **104** signals the application **105** (FIG. 2) to actuate the model toy **103** (FIG. 2). If there is an existing queue, the web browser extension **118** (FIG. 1) adds a tip reaction to queue **308.** The user can continue chatting with the model and tip the model until the end of the chat session **309.** In this regard, either the user and/or the model can end the chat session **310,** or the chat session may be automatically ended after a predetermined period of time.

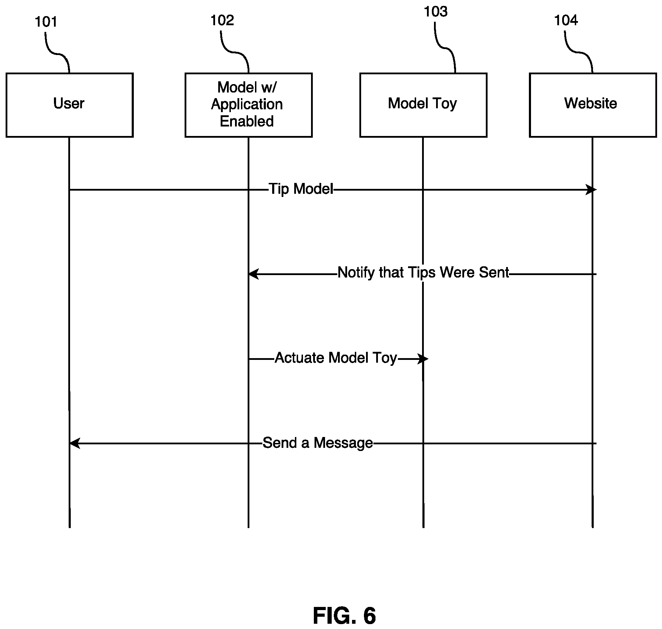

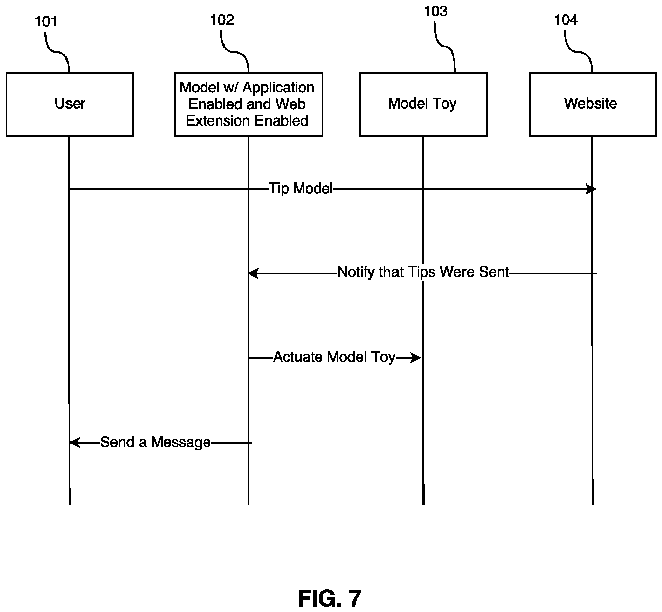

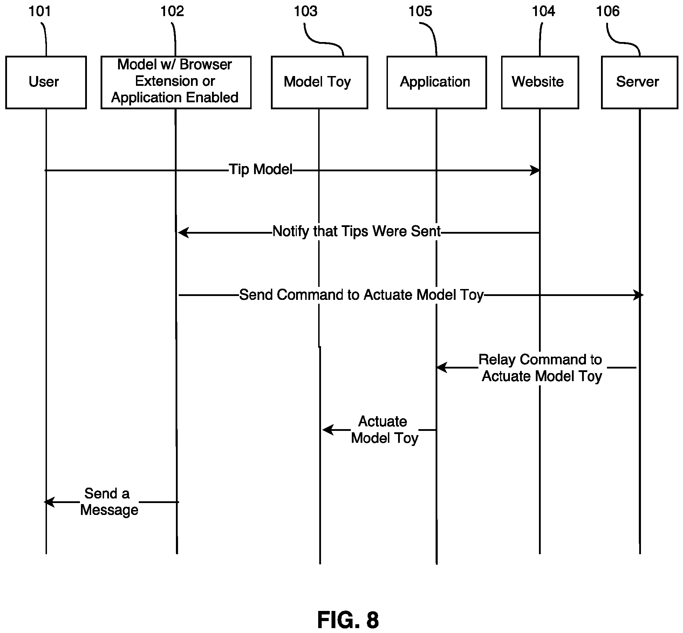

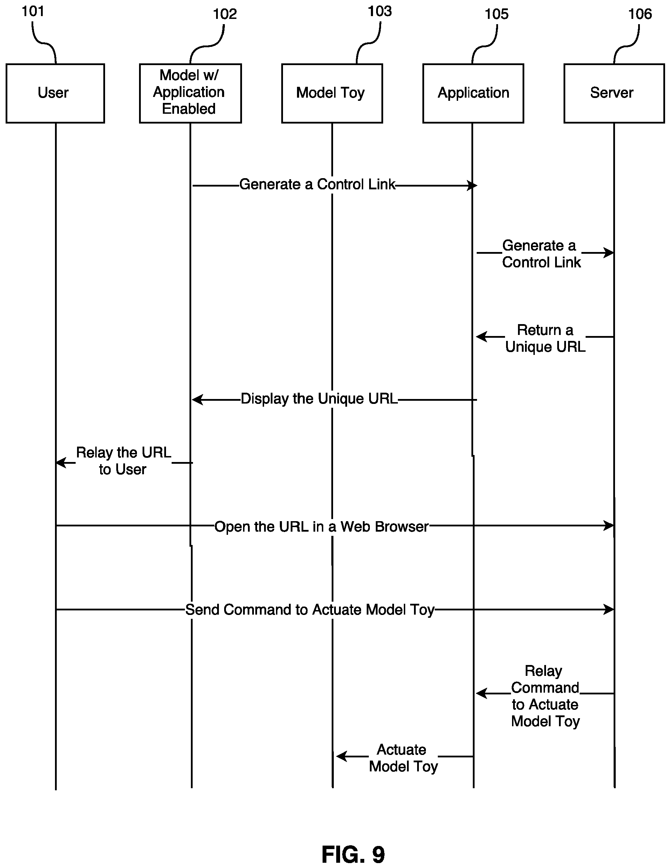

References are now made to **FIGs. 6** through **9****,** which show exemplary diagrams of various embodiments of the present invention. In some embodiments, the user **101** can tip the model via the website **104,** for example, by inputting the tip amount in a chat room. The model's UI or the model toy is notified of the tip via the web browser extension or the website. When the tip amount is validated and the model toy **103** is actuated in accordance with the model's tip parameters, the user can receive an automated message via the website **104,** wherein the message comprises a thank you message or a predetermined message customized by the model 102.

In some embodiments, the model 102 may operate a mode! device having an application installed thereon, wherein the application is configured to automatically actuate the model toy 103 when it receives notification that tips were sent to the model via the website 104

In some embodiments, the present system further comprises a server 106, wherein the model, via the application enabled on the model device, is configured to send command to the server 106 to actuate the model toy 103, and further wherein the server 106 is configured to relay the command to actuate the model toy 103. When the tip amount is validated, the web browser extension is configured to send an automated message to the user 101 as described above.

In a system according to the invention, the model 102 can generate a live control link via the application 105, which triggers the server 106 to output a unique URL via the browser or the website and relay it back to the application 105 to display it on its UI. The model 102 can relay the URL (i.e., the live control link) to the user 101. The user can open the URL in a web browser and access a control panel to control the model toy. The user is not required to tip the model when using the control panel. When the user enters a command to actuate the model toy, the server 106 relays the command back to the application 105 in order to actuate the model toy.

The following list of events may be considered a more concrete example of the functionality of the present system and method, in a realistic scenario involving a user and a model.
I. Step 1: The model defines tipping parameters as follows:
   a. 1 token: vibrate a model toy at a low speed for 2 seconds
   b. 3-5 tokens: vibrate a model toy at a low speed and rotate a model toy at a medium speed for 4 seconds
II. Step 2: A user and a model enter into an online chat room (e.g., on a website, a web application, and the like) to begin a chat session.
   a. The model turns on the model toy by actuating the toy's control buttons or via the application installed on the model device (e.g., computer, mobile phone, etc.) and/or enables the UI to scan the chat sessions for tips.
III. Step 3: The user views the model's tipping parameters and tips the model 1 token by inputting the tip amount in the conversation thread during the chat session.
IV. Step 4: The tip amount is automatically detected via a web browser extension and analyzed to determine whether the tip amount falls within the model's tipping parameters.
V. Step 5: The web browser extension validates the tip amount and determines the corresponding act based on the tip amount.
VI. Step 6: The server transmits command signal to vibrate the model toy at a low speed for 2 seconds, either directly to the model toy or to the application, wherein the application is configured to relay the command signal to the model toy to actuate the same.
VII. Step 7: The model sends a thank you message to the user.

The following list of events may be considered a more concrete example of the functionality of the present system and method, in a realistic scenario involving a live control link:
I. Step 1: A model generates a live control link using a link generator of a web browser extension or a website.
   a. The model can define the duration for which a user can use the live control feature (unlimited amount of time or a limited amount of time).
II. Step 2: The model transmits the live control link to one or more users.
III. Step 3: A user accesses the live control link and a virtual control panel pops up on UI.

## Claims

1. A system for providing online communication, in particular during an online chat session, comprising:
a) an operable adult toy (103) configured to receive signals, wherein the adult toy (103) is a model toy (103);
b) a memory (110) having stored thereon instructions (111);
c) a processor (109) to execute said instructions (111) resulting in a software application;
d) said software application configured to:
d1) before the start of the online chat session, define input parameters, wherein said input parameters comprise one or more ranges of input values and actions correlating to each of said one or more ranges, wherein the actions mean an operation of the adult toy (103), for example vibrating, rotating, thrusting or oscillating;
d2) wherein the input parameters are tip parameters and wherein the input values are tip amounts,
d3) scan for inputs during the online chat session and receive an input from one or more users,
d4) wherein said input is a tip and wherein said tip comprises virtual currency;
d5) determine whether said input falls within said one or more ranges; and
d6) if said input falls within said input parameters, actuating said adult toy (103) in accordance with the input value,
**characterized in that**
e) said software application comprises a link generator for generating a live control link, further wherein said live control link comprises a unique URL, wherein opening the unique URL gives a user (101) access to controlling the adult toy (103),
f) wherein the system comprises a server (106),
g) wherein the system is configured such that
- the model (102) can generate the live control link via the application (105), which triggers the server (106) to output the unique URL and relay it back to the application (105) to display it on its UI (125),
- the model (102) can relay the URL to the user (101),
- the user (101) can open the URL in a web browser and access a control panel to control the model toy (103), wherein when the user (101) enters a command to actuate the model toy (103), the server (106) relays the command back to the application (105) in order to actuate the model toy (103).

2. The system of claim 1, wherein said adult toy (103) is Wi-Fi-enabled or Bluetooth-enabled; and said software application configured to actuate said adult toy (103).

3. The system of claim 1, further comprising:
a model device (115, 115A, 115B) having the application (105) installed thereon, wherein said application (105) is configured to send commands to said adult toy (103) to actuate the same.

4. The system of claim 1, wherein said link generator is configured to invalidate previously generated live control link.

5. The system of claim 1, wherein said link generator is configured to build a queue of multiple links.

6. A system according to any of the previous claims, comprising:
one or more user devices (114, 114A, 114B) and a model device (115, 115A, 115B) in connection with a network (112);
each of said one or more user devices (114, 114A, 114B) and said model device (115, 115A, 115B) having a web browser (121) thereon for accessing a website, said website providing an online chat room;
wherein the adult toy (103) is in communication with said model device (115, 115A, 115B);
said web browser comprising said software application.

7. The system of claim 6, wherein said adult toy (103) is Wi-Fi-enabled or Bluetooth-enabled; and said software application configured to actuate said adult toy (103).

8. The system of claim 6, wherein said model device (115, 115A, 115B) comprises an application (105) installed thereon;
said application (105) configured to send commands to said adult toy to actuate said adult toy (103).

9. The system of claim 6, wherein said software application comprises a link generator for generating a live control link, further wherein said live control link comprises a unique URL.

10. The system of claim 9, wherein said link generator is configured to invalidate previously generated live control link.

11. The system of claim 9, wherein said link generator is configured to build a queue of multiple links.

12. A computer based method for providing adult entertainment online by means of a system according to any of the previous claims, comprising the steps of:
defining the input parameters, wherein said input parameters comprise one or more ranges of input values and actions correlating to each of said one or more ranges;
activating the adult toy (103) in communication with a model device (115);
starting a chat session with one or more users (101) and a model (102) via a website;
receiving an input from said one or more users (101), wherein the input is a tip and wherein said tip comprises virtual currency;
determining whether said input falls within said input parameters;
if said input falls within said input parameters, actuating said adult toy (103) in accordance with the input value;
**characterized in that** the method furthermore comprises the steps of:
generating the live control link via the link generator, wherein said live control link comprises the unique URL, wherein opening the unique URL gives the user (101) access to controlling the adult toy (103),
- wherein the model (102) generates the live control link via the application (105), which triggers the server (106) to output the unique URL and relay it back to the application (105) to display it on its UI (125),
- wherein the model (102) relays the URL to the user (101),
- wherein the user (101) opens the URL in the web browser and accesses the control panel to control the model toy (103), wherein when the user (101) enters a command to actuate the model toy (103), the server (106) relays the command back to the application (105) in order to actuate the model toy (103).

13. The method of claim 12, wherein said adult toy (103) is Wi-Fi-enabled or Bluetooth-enabled; said adult toy (103) configured to receive command signals from the server (106).

14. The method of claim 12, wherein said model device (115, 115A, 115B) comprises the application (105) installed thereon;
said application (105) configured to send commands to said adult toy (103) to actuate said adult toy (103).

15. The method of claim 12, further comprising the steps of:
invalidating previously generated live control link via said link generator.

16. The method of claim 12, wherein said link generator is configured to build a queue of multiple links.

## Patentansprüche

1. System zum Bereitstellen von Online-Kommunikation, insbesondere während einer Online-Chat-Session, umfassend:
a) ein in Betrieb setzbares Erwachsenenspielzeug (103) eingerichtet zum Empfangen von Signalen; wobei das Erwachsenenspielzeug (103) ein Model-Spielzeug (103) ist;
b) einen Speicher (110), auf welchem Instruktionen (111) gespeichert sind;
c) einen Prozessor (109) um die genannten Instruktionen (111) resultierend in einer Softwareanwendung auszuführen;
d) wobei besagte Softwareanwendung eingerichtet ist um:
d1) vor dem Start der Online-Chat-Session Eingabeparameter zu definieren, wobei besagte Eingabeparameter einen oder mehr Bereiche von Eingangswerten und Aktionen, welche jedem der besagten ein oder mehr Bereiche zugeordnet sind, umfassen, wobei die Aktionen eine Inbetriebsetzung des Erwachsenenspielzeugs (103) bedeuten, zum Beispiel Vibrieren, Rotieren, Drücken oder Oszillieren;
d2) wobei die Eingabeparameter Trinkgeld-Parameter sind und wobei die Eingangswerte Trinkgeld-Werte sind,
d3) während der Online-Chat-Session auf Eingaben zu prüfen und eine Eingabe von einem oder mehreren Nutzern zu empfangen,
d4) wobei besagte Eingabe ein Trinkgeld ist und wobei besagtes Trinkgeld eine virtuelle Währung umfasst;
d5) zu ermitteln, ob besagte Eingabe in einen oder mehrere der besagten Bereiche fällt; und
d6) wenn besagte Eingabe innerhalb besagte Eingabeparameter fällt, besagtes Erwachsenenspielzeug (103) entsprechend dem Eingangswert zu aktivieren,
**dadurch gekennzeichnet, dass**
e) besagte Softwareanwendung einen Link-Generator zum Erzeugen eines Live-Kontroll-Links umfasst, wobei besagter Live-Kontroll-Link ferner eine einmalige URL umfasst, wobei ein Öffnen der einmaligen URL einem Nutzer (101) Zugang zum Kontrollieren des Erwachsenenspielzeug (103) gibt,
f) wobei das System einen Server (106) umfasst,
g) wobei das System derart konfiguriert ist, dass
- das Model (102) den Live-Kontroll-Link über die Anwendung (105) generieren kann, welche den Server (106) veranlasst, die einmalige URL auszugeben und sie an die Anwendung (105) zurück zu übermitteln um sie auf ihrer Benutzerschnittstelle (125) anzuzeigen,
- das Model (102) die URL an den Nutzer (101) übermitteln kann,
- der Nutzer (101) die URL in einem Webbrowser öffnen kann und auf ein Bedienfeld zugreifen kann, um das Model-Spielzeug (103) zu kontrollieren, wobei wenn der Nutzer (101) einen Befehl zum Aktuieren des Model-Spielzeugs (103) eingibt, der Server (106) den Befehl an die Anwendung (105) zurück übermittelt um das Model-Spielzeug (103) zu aktuieren.

2. System nach Anspruch 1, wobei besagtes Erwachsenenspielzeug (103) Wi-Fi-fähig oder Bluetooth-fähig ist; und besagte Softwareanwendung eingerichtet ist um besagtes Erwachsenenspielzeug (103) zu aktuieren.

3. System nach Anspruch 1, ferner umfassend:
ein Modelgerät (115, 115A, 115B), auf welchem die Anwendung (105) installiert ist, wobei besagte Anwendung (105) konfiguriert ist, um Befehle an besagtes Erwachsenenspielzeug (103) zu senden um selbiges zu aktuieren.

4. System nach Anspruch 1, wobei besagter Link-Generator konfiguriert ist, um zuvor generierten Live-Kontroll-Link zu annullieren.

5. System nach Anspruch 1, wobei besagter Link-Generator konfiguriert ist, um eine Schlange von mehreren Links aufzubauen.

6. System nach einem der vorhergehenden Ansprüche, umfassend:
ein oder mehrere Nutzergeräte (114, 114A, 114B) und ein Modelgerät (115, 115A, 115B) in Verbindung mit einem Netzwerk (112);
jedes der besagten ein oder mehren Nutzergeräte (114, 114A, 114B) und besagtes Modelgerät (115, 115A, 115B) umfassend einen Webbrowser (121) auf sich um eine Webseite zu betreten, wobei besagte Webseite einen Online-Chatraum zur Verfügung stellt;
wobei das Erwachsenenspielzeug (103) in Kommunikation mit besagtem Modelgerät (115, 115A, 115B) ist;
wobei besagter Webbrowser besagte Softwareanwendung umfasst.

7. System nach Anspruch 6, wobei besagtes Erwachsenenspielzeug (103) Wi-Fi-fähig oder Bluetooth-fähig ist; und besagte Softwareanwendung konfiguriert ist um besagtes Erwachsenenspielzeug (103) zu aktuieren.

8. System nach Anspruch 6, wobei besagtes Modelgerät (115, 115A, 115B) eine Anwendung (105) auf sich installiert umfasst;
wobei besagte Anwendung (105) konfiguriert ist, um Befehle an besagtes Erwachsenenspielzeug zu senden um besagtes Erwachsenenspielzeug (103) zu aktuieren.

9. System nach Anspruch 6, wobei besagte Softwareanwendung einen Link-Generator zum Generieren eines Live-Kontroll-Links umfasst, wobei besagter Live-Kontroll-Link ferner eine einmalige URL umfasst.

10. System nach Anspruch 9, wobei besagter Link-Generator konfiguriert ist, um zuvor generierten Live-Kontroll-Link zu annullieren.

11. System nach Anspruch 9, wobei besagter Link-Generator konfiguriert ist, um eine Schlange von mehreren Links aufzubauen.

12. Computer-basiertes Verfahren zum Online-Bereitstellen von Erwachsenenunterhaltung mittels eines Systems nach einem der vorhergehenden Ansprüche, umfassend die Schritte:
die Eingabeparameter definieren, wobei besagte Eingabeparameter einen oder mehrere Bereiche von Eingangswerten und Aktionen, welche jedem der besagten einen oder mehreren Eingangsbereiche entsprechen, umfassen;
Aktivieren des Erwachsenenspielzeug (103), in Kommunikation mit dem Modelgerät (115);
Starten einer Chat-Session mit einem oder mehreren Nutzern (101) und einem Model (102) über eine Webseite;
Empfangen einer Eingabe von besagtem einen oder mehreren Nutzern (101), wobei die Eingabe ein Trinkgeld ist und wobei besagtes Trinkgeld eine virtuelle Währung umfasst;
Überprüfen, ob besagte Eingabe in besagte Eingabeparameter fällt;
wenn besagte Eingabe in besagte Eingabeparameter fällt, Aktuieren des besagten Erwachsenenspielzeugs (103) entsprechend dem Eingangswert;
**dadurch gekennzeichnet, dass** das Verfahren ferner die folgenden Schritte umfasst:
Generieren des Live-Kontroll-Links über den Link-Generator, wobei besagter Live-Kontroll-Link die einmalige URL umfasst, wobei ein Öffnen der einmaligen URL dem Nutzer (101) Zugang zum Kontrollieren des Erwachsenenspielzeugs (103) gibt,
- wobei das Model (102) den Live-Kontroll-Link über die Anwendung (105) generiert, welche den Server (106) veranlasst, die einmalige URL auszugeben und sie an die Anwendung (105) zurück zu übermitteln um sie auf ihrer Benutzerschnittstelle (125) anzuzeigen,
- wobei das Model (102) die URL an den Nutzer (101) ünermittelt,
- wobei der Nutzer (101) die URL in dem Webbrowser öffnet und auf das Bedienfeld zugreift um das Model-Spielzeug (103) zu kontrollieren, wobei wenn der Nutzer (101) einen Befehl eingibt, um das Model-Spielzeug (103) zu aktuieren der Server (106) den Befehl an die Anwendung (105) zurück übermittelt, um das Model-Spielzeug (103) zu aktuieren.

13. Verfahren nach Anspruch 12, wobei besagtes Erwachsenenspielzeug (103) Wi-Fi-fähig oder Bluetooth-fähig ist; wobei besagtes Erwachsenenspielzeug (103) konfiguriert ist um Befehlssignale von dem Server (106) zu empfangen.

14. Verfahren nach Anspruch 12, wobei besagtes Modelgerät (115, 115A, 115B) die Anwendung (105) darauf installiert umfasst;
wobei besagte Anwendung (105) konfiguriert ist um Befehle an besagtes Erwachsenenspielzeug (103) zu senden um besagtes Erwachsenenspielzeug (103) zu aktuieren.

15. Verfahren nach Anspruch 12, ferner umfassend die Schritte:
den zuvor erzeugten Live-Kontroll-Link über besagten Link-Generator annullieren.

16. Verfahren nach Anspruch 12, wobei besagter Link-Generator konfiguriert ist, um eine Schlange von mehreren Links aufzubauen.

## Revendications

1. Système pour fournir de la communication en ligne, notamment lors d'une session de chat en ligne, comprenant:
a) un jouet adulte (103) utilisable configuré pour recevoir des signaux, où le jouet adulte (103) est un jouet mannequin (103);
b) une mémoire (110) sur laquelle sont stockées des instructions (111);
c) un processeur (109) pour exécuter lesdites instructions (111) aboutissant à une application logicielle;
d) ladite application logicielle configure pour:
d1) avant le début de la session de chat en ligne, définir des paramètres d'entrée, où lesdits paramètres d'entrée comprennent une ou plusieurs plages de valeurs d'entrée et des actions en corrélation avec chacune desdites une ou plusieurs plages, où les actions signifient une utilisation du jouet adulte (103), par exemple vibrer, tourner, pousser ou osciller;
d2) où les paramètres d'entrée sont des paramètres de pourboire et où les valeurs d'entrée sont des montants de pourboire,
d3) rechercher des entrées pendant la session de chat en ligne et recevoir une entrée d'un ou plusieurs utilisateurs,
d4) où ladite entrée est un pourboire et où ledit pourboire comprend une monnaie virtuelle ;
d5) déterminer si ladite entrée se situe dans ladite une ou plusieurs plages; et
d6) si ladite entrée se situe dans lesdits paramètres d'entrée, actionner ledit jouet adulte (103) conformément à la valeur d'entrée,
**caractérisé en ce que**
e) ladite application logicielle comprend un générateur de lien pour générer un lien de contrôle en direct, où en outre ledit lien de contrôle en direct comprend une URL unique, où l'ouverture de l'URL unique donne à un utilisateur (101) accès au contrôle du jouet adulte (103),
f) où le système comprend un serveur (106),
g) où le système est configuré de telle sorte que
- le mannequin (102) peut générer le lien de contrôle en direct via l'application (105), ce qui déclenche le serveur (106) pour produire l'URL unique et la relayer en arrière à l'application (105) pour l'afficher sur son interface utilisateur (125),
- le mannequin (102) peut relayer l'URL à l'utilisateur (101),
- l'utilisateur (101) peut ouvrir l'URL dans un navigateur web et accéder à un panneau de contrôle pour commander le jouet mannequin (103), où lorsque l'utilisateur (101) entre une commande pour actionner le jouet mannequin (103), le serveur (106) relaie la commande en arrière à l'application (105) afin d'actionner le jouet mannequin (103).

2. Système selon la revendication 1, où ledit jouet adulte (103) est compatible avec le Wi-Fi ou le Bluetooth; et ladite application logicielle est configurée pour actionner ledit jouet adulte (103).

3. Système selon la revendication 1, comprenant en outre:
un dispositif mannequin (115, 115A, 115B) sur lequel est installée l'application (105), où ladite application (105) est configurée pour envoyer des commandes audit jouet adulte (103) pour actionner celui-ci.

4. Système selon la revendication 1, où ledit générateur de lien est configuré pour invalider le lien de contrôle en direct précédemment généré.

5. Système selon la revendication 1, où ledit générateur de lien est configuré pour construire une file d'attente de liens multiples.

6. Système selon l'une quelconque des revendications précédentes, comprenant:
un ou plusieurs dispositifs utilisateurs (114, 114A, 114B) et un dispositif mannequin (115, 115A, 115B) en connexion avec un réseau (112);
chacun desdits un ou plusieurs dispositifs utilisateur (114, 114A, 114B) et ledit dispositif mannequin (115, 115A, 115B) ayant un navigateur web (121) sur lui pour accéder à un site web, ledit site web fournissant un salon de chat en ligne;
où le jouet adulte (103) est en communication avec ledit dispositif mannequin (115, 115A, 115B);
ledit navigateur web comprenant ladite application logicielle.

7. Système selon la revendication 6, où ledit jouet adulte (103) est compatible avec le Wi-Fi ou le Bluetooth; et ladite application logicielle est configurée pour actionner ledit jouet adulte (103).

8. Système selon la revendication 6, où ledit dispositif mannequin (115, 115A, 115B) comprend une application (105) installée sur celui-ci ;
ladite application (105) étant configurée pour envoyer des commandes audit jouet adulte afin d'actionner ledit jouet adulte (103).

9. Système selon la revendication 6, où ladite application logicielle comprend un générateur de lien pour générer un lien de contrôle en direct, où en outre ledit lien de contrôle en direct comprend une URL unique.

10. Système selon la revendication 9, où ledit générateur de lien est configuré pour invalider le lien de contrôle en direct précédemment généré.

11. Système selon la revendication 9, où ledit générateur de lien est configuré pour construire une file d'attente de liens multiples.

12. Procédé informatique pour fournir des divertissements pour adultes en ligne au moyen d'un système selon l'une quelconque des revendications précédentes, comprenant les étapes suivantes:
définir les paramètres d'entrée, où lesdits paramètres d'entrée comprennent une ou plusieurs plages de valeurs d'entrée et des actions en corrélation avec chacune desdites une ou plusieurs plages;
activer le jouet adulte (103) en communication avec un dispositif mannequin (115);
démarrer une session de chat avec un ou plusieurs utilisateurs (101) et un mannequin (102) via un site web;
recevoir une entrée de la part desdits un ou plusieurs utilisateurs (101), où l'entrée est un pourboire et où ledit pourboire comprend une monnaie virtuelle;
déterminer si ladite entrée se situe dans lesdits paramètres d'entrée ;
si ladite entrée se situe dans lesdits paramètres d'entrée, actionner ledit jouet adulte (103) conformément à la valeur d'entrée ;
**caractérisé en ce que** la méthode comprend en outre les étapes suivantes:
générer le lien de contrôle en direct via le générateur de lien, où ledit lien de contrôle en direct comprend l'URL unique, où l'ouverture de l'URL unique donne à l'utilisateur (101) l'accès au contrôle du jouet adulte (103),
- où le mannequin (102) génère le lien de contrôle en direct via l'application (105), ce qui déclenche le serveur (106) pour produire l'URL unique et la relayer en arrière à l'application (105) pour l'afficher sur son interface utilisateur (125),
- où le mannequin (102) relaie l'URL à l'utilisateur (101),
- où l'utilisateur (101) ouvre l'URL dans le navigateur web et accède au panneau de contrôle pour commander le jouet mannequin (103), où lorsque l'utilisateur (101) entre une commande pour actionner le jouet mannequin (103), le serveur (106) relaie la commande en arrière à l'application (105) afin d'actionner le jouet mannequin (103).

13. Procédé selon la revendication 12, où ledit jouet adulte (103) est compatible avec le Wi-Fi ou le Bluetooth; ledit jouet adulte (103) étant configuré pour recevoir des signaux de commande du serveur (106).

14. Procédé selon la revendication 12, où ledit dispositif mannequin (115, 115A, 115B) comprend l'application (105) installée sur lui;
ladite application (105) étant configurée pour envoyer des commandes audit jouet adulte (103) pour actionner ledit jouet adulte (103).

15. Procédé selon la revendication 12, comprenant en outre les étapes de:
invalider le lien de contrôle en direct précédemment généré par ledit générateur de lien.

16. Procédé selon la revendication 12, où ledit générateur de lien est configuré pour construire une file d'attente de liens multiples.
